# EUROPEAN PATENT APPLICATION

(11) **EP 2 359 813 A1**
(43) Date of publication of application: **24.08.2011**
(21) Application number: 10152690.3
(22) Date of filing: 04.02.2010
(51) Int. Cl.: A61K 9/20, A61K 31/506

(54) **Pharmaceutical composition comprising N-(2-chloro-6-methylphenyl)-2-[[6-[4-(2-hydroxyethyl)-1-piperazinyl]-2-methyl-4-pyrimidinyl]amino]-5-thiazolecarboxamid**

(71) Applicant: Ratiopharm GmbH, 89079 Ulm (DE)
(72) Inventor: Stefan, Ralph, 88370, Ebenweiler (DE); Stumm, Daniela, 83730, Fischbachau (DE)
(74) Representative: Teipel, Stephan

(57) **Abstract**

The present invention relates to a pharmaceutical composition comprising Dasatinib as active pharmaceutical ingredient.

## Description

### Field of the invention

The present invention relates to a pharmaceutical composition comprising N-(2-chloro-6-methylphenyl)-2-[[6-[4-(2-hydroxyethyl)-1-piperazinyl]-2-methyl-4-pyrimidinyl]amino]-5-thiazolecarboxamid as active pharmaceutical ingredient.

### Background of the invention

The invention relates to a pharmaceutical composition comprising N-(2-chloro-6-methylphenyl)-2-[[6-[4-(2-hydroxyethyl)-1-piperazinyl]-2-methyl-4-pyrimidinyl]amino]-5-thiazolecarboxamid and a process of preparing said composition.

The compound N-(2-chloro-6-methylphenyl)-2-[[6-[4-(2-hydroxyethyl)-1-piperazinyl]-2-methyl-4-pyrimidinyl]amino]-5-thiazolecarboxamid (INN: Dasatinib) is a small molecule tyrosine kinase inhibitor which is used to treat disorders like chronic myelogenous leukemia (CML) and acute lymphoblastic leukemia (ALL). Its activity relies on the inhibition of cellular signalling by targeting multiple receptor and cellular tyrosine kinases including Bcr-Abl, Src, cKit and platelet-derived growth factor receptor β (PDGFRβ). Since these kinases activate a number of downstream effectors and mediate a large number of developmental, cell proliferation and cell differentiation pathways, the simultaneous inhibition of these targets can achieve normalization or at least reduction of malignant blood cell counts, even in lmatinib resistant patients.

Dasatinib and its pharmaceutical effects on immunologic and oncologic disorders are described in WO 00/62778. Further medical uses of Dasatinib and its salts are inter alia known from WO 2006/135790 and WO 2007/047893.

WO 00/62778 discloses a process of preparing Dasatinib. According to this and other known manufacturing processes (e.g. WO 2005/077945), Dasatinib is obtained as a solid. WO 2007/035874 describes pharmaceutical formulations for oral administration, combining Dasatinib with pH modifying agents.

WO 2006/121742 discloses conventional pharmaceutical formulations comprising Dasatinib intended for oral administration. According to the examples Dasatinib tablets are obtained by wet granulation.

Typically, Dasatinib is administered orally in a dose of 70 mg twice daily, which, if necessary, has to be varied according to individual tolerance and safety. Thus, in order to obtain sufficiently flexible dosing, individual dosage forms generally contain 20, 50 and 70 mg of Dasatinib. Film coated tablets comprising the crystalline monohydrate of Dasatinib are sold under the brand name Sprycel® (by Bristol Myers Squibb). This crystalline monohydrate of Dasatinib is described in WO 2005/077945.

It is an object of the invention to provide a pharmaceutical composition in the form of a tablet, comprising Dasatinib, which tablet can be easier and more cost efficient prepared than the known formulations. At the same time, the new composition should warrant good solubility and bioavailability of the active pharmaceutical ingredient. Further, the composition should possess good storage stability, and homogeneity.

It has now surprisingly been found that the wet granulation required for the prior art preparation of Dasatinib containing tablets can be avoided if Dasatinib is employed in its salt free anhydrate form as described in WO 2005/077945, in form of its hydrochloride salt as described in J. Med. Chem. 2004, 47, 6658-6661 or in form of its hemi ethanol solvate as disclosed in US 2006/0004067 (example 10). If these particular forms of Dasatinib are employed, it is possible to prepare tablets by direct compression.

Without the intention to being bound to any theory, it is believed that the above certain forms of Dasatinib exhibit particular characteristics of the surfaces of their particles, such as crystal form, relative dimensions of the surfaces, aspect ratio, etc., making them suitable for direct compression methods. In contrast thereto, Dasatinib hydrate used in the prior art tablets is unsuitable for direct compression methods.

Thus, the present invention relates to a pharmaceutical composition in the form of a tablet comprising Dasatinib as active pharmaceutical ingredient, wherein the active pharmaceutical ingredient is present in its salt free anhydrate form, in form of its hydrochloride salt or in form of its hemi ethanol solvate, wherein the tablet is obtainable by direct compression.

N-(2-Chloro-6-methylphenyl)-2-[[6-[4-(2-hydroxyethyl)-1-piperazinyl]-2-methyl-4-pyrimidinyl]amino]-5-thiazolecarboxamid (Dasatinib) has the following chemical structure:

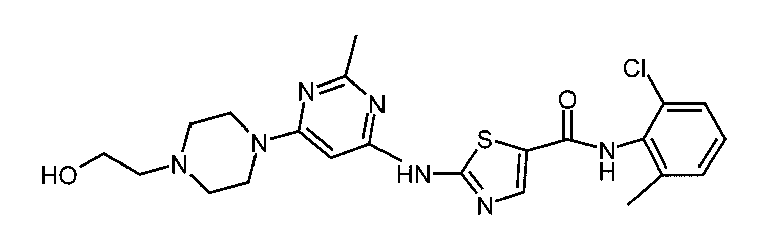

Dasatinib as well as its salts can be readily synthesized using techniques well known in the art. The synthesis of crystalline Dasatinib anhydrate is disclosed in WO 2005/077945. The crystalline hydrochloride salt as used for the present invention can be prepared according to the procedure disclosed in J. Med. Chem. 2004, 47, 6658-6661. The synthesis of Dasatinib hemi ethanol solvate is disclosed in US 2006/0004067.

The term "crystalline" refers to any non amorphous forms of the active pharmaceutical ingredient. The term "amorphous form" refers to a form of active pharmaceutical ingredient which has no long-range order like crystalline forms. The atoms or molecules of a material present in amorphous form are arranged in a non-uniform array. It is for example possible to distinguish amorphous from crystalline forms of a compound by powder X-ray diffraction.

The term "pharmaceutical composition" refers to a single dosage form, namely a tablet. Where it is referred to the total weight of the pharmaceutical composition the total weight is the weight of the tablet excluding, if applicable, the weight of any coating.

The term "Dasatinib anhydrate" refers to Dasatinib in its salt free anhydrate form. Preferably the anhydrate form is crystalline.

The term "Dasatinib hydrochloride" refers to Dasatinib monohydrochloride,. Preferably the hydrochloride form is crystalline. Also preferably the hydrochloride is a hemi hydrate.

The term "Dasatinib hemi ethanol solvate" refers to Dasatinib in its salt free hemi ethanol solvate form. Preferably the hemi ethanol solvate is crystalline.

The term "active pharmaceutical ingredient " (API) refers to Dasatinib in its anhydrate form, in its hydrochloride form, or in its hemi ethanol solvate form.

The pharmaceutical composition of the present invention preferably comprises 20 +/-2 mg, 50 +/-5 mg or 70 +/-7 mg active pharmaceutical ingredient based on the weight of the free base in its unsolvated form.

The active pharmaceutical ingredient is preferably present in the pharmaceutical composition in an amount of 5 to 70 wt.%, more preferably 10 to 50 wt.% and most preferably 20 to 40 wt.%, wherein the respective amounts are being referred to the total weight of the composition.

Advantageous properties with respect to solubility, homogeneity, stability, flowability, compressibility and the avoidance of demixing tendencies are achieved if the active pharmaceutical ingredient of the present invention is provided in a mean particle size of 1 to 300 µm, preferably 1 to 100, more preferably 5 to 50 µm. The particle size distribution can be determined via laser scattering performed on the API dispersed in an inert suspending medium.

Since the active pharmaceutical ingredient as obtained from the manufacturing process may vary in its particle size, it might have to be milled, grinded and/or sieved in order to warrant the uniformity of the suitable particle size.

The grinding can for example be performed using pin or hammer mills. By varying the rotation speed of the mill, the influent of the product and/or the duration of milling, particles possessing the desired particle size and/or specific surface area can be obtained. The application of an air jet mill might be advantageous if a particle size of e.g. less than 10 µm is required. In any case, the API particle size can also be adjusted by mixing APIs of different particle sizes and/or screening with sieves of suitable mesh sizes.

A bulk density of the mixture of ingredients of the pharmaceutical composition prior to direct compression ranging from of 0.3 to 0.9 g/ml, preferably of 0.4 to 0.8 g/ml is advantageous.

The mixture of ingredients of the pharmaceutical composition of the invention prior to direct compression preferably possesses a Hausner factor in the range of 1.05 to 1.65, more preferably of 1.1 to 1.5. The Hausner factor is the ratio of bulk density to tapped density.

Due to the advantageous properties of the active pharmaceutical ingredient and the manufacturing process of the pharmaceutical composition comprising the latter, the pharmaceutical composition of the present invention possesses excellent solubility, homogeneity, flowability and compressibility. Further, it shows convenient workability over the whole range of suitable concentrations of the active pharmaceutical ingredient within the composition.

The pharmaceutical composition of the present invention may further comprise one or more pharmaceutically acceptable excipients, such as fillers, binding agents, lubricants, glidants, antisticking agents, solubility enhancers and disintegrating agents. As pharmaceutically acceptable excipients conventional excipients known to the person skilled in the art may be used. See for example "Lexikon der Hilfsstoffe für Pharmazie, Kosmetik und angrenzende Gebiete", edited by H. P. Fiedler, 4th Edition, Edito Cantor, Aulendorf and earlier editions, and "Handbook of Pharmaceutical Excipients", Third Edition, edited by Arthur H. Kibbe, American Pharmaceutical Association, Washington, USA, and Pharmaceutical Press, London.

Preferred examples of the fillers are lactose, mannitol, sorbitol, isomalt or microcrystalline cellulose. The filler is suitably present in an amount of about 10 to 80 wt.%, preferably of about 30 to 70 wt.% of the total weight of the composition.

The binding agent can for example be microcrystalline cellulose (MCC), hydroxypropylmethyl cellulose (HPMC), isomalt or maize starch. Preferably the binding agent is present in an amount of about 1 to 25 wt.%, more preferably at about 2 to 15 wt.% of the total weight of the composition.

The lubricant is preferably a stearate, more preferably an earth alkali metal stearate, such as magnesium stearate. The lubricant is suitably present in an amount of about 0.1 to 2 wt.%, preferably about 0.5 to 1.5 wt.% of the total weight of the composition.

Preferred disintegrating agents are croscarmellose sodium, sodium carboxymethyl starch or cross-linked polyvinylpyrrolidone (crospovidone). The disintegrating agent is suitably present in an amount of about 0.1 to 20 wt.%, preferably at about 1 to 10 wt.% of the total weight of the composition.

The glidant can for example be colloidal silicon dioxide. Preferably the binding agent is present in an amount of about 0.5 to 8 wt.%, more preferably at about 0.5 to 3 wt.% of the total weight of the composition.

The antisticking agent is for example talcum and may be present in an amount of about 1 to 5 %.wt, more preferably in an amount of about 1.5 to 3 wt.% of the total weight of the composition.

An improvement of the solubility of the active pharmaceutical ingredient can for example be achieved by the addition of complex forming agents/compounds (e.g. sodium benzoate, sodium salicylate or cyclodextrins), alternation of solvent properties (e.g. by adding PVP or polyethylene glycols) or the addition of solubilizers which form tenside micelles (e.g. surfactants).

Suitable solubilizers are for example surfactants such as polyoxyethylene alcohol ethers (e.g. Brij®), polysorbates (e.g. Tween®) or polyoxypropylene polyoxyethylene copolymers (poloxamer; e.g. Pluronic®). Solubilizers may be present in an amount of about 0.5 to 7 %.wt, more preferably of about 1 to 5 %.wt of the total weight of the composition.

Alternatively, a pseudo-emulsifier can be used. Its mechanism of action mainly relies on an enhancement of viscosity. However, pseudo-emulsifiers also possess emulsifying properties. Preferred pseudo-emulsifiers are for example cellulose ethers, gum Arabic or tragacanth. Pseudo-emulsifiers may be present in amounts of about 1 to 10 %wt., more preferably of about 3 to 7 %.wt of the total weight of the composition.

The pharmaceutical composition of the present invention is formulated as a tablet. A particularly preferred pharmaceutical composition is in the form of a film coated tablet. The tablets can be obtained by direct compression of a suitable powder mixture, i.e. without any preceding granulation of the excipients.

The tablets may further be coated either with any functional or non functional film building agent such as cellulose derivatives, polyacrylates, polymethacrylates, vinyl polymers and shellac or any ready to use film building composition known in the art, e.g. selected from the Eudragit™ or Pharmacoat™ series depending on the desired effect. The non functional film coat Pharmacoat™ 603 is especially preferred.

The pharmaceutical composition may contain dosage amounts of 20 ± 2mg, 50 ± 5 mg and 70 ± 7 mg of Dasatinib (referred to the weight of the free base in anhydrous form). Thus the administered amount can be readily varied according to individual tolerance and safety warranting more flexible dosing than the standard dose of 70 mg once daily.

In a preferred embodiment the pharmaceutical composition of the present invention comprises 20 to 30 wt.% of the active pharmaceutical ingredient, 60 to 70 wt.% of a spray dried mixture of lactose monohydrate and maize starch, 5 to 6 wt.% crosslinked polyvinylpyrrolidone, 1 to 1.5 wt.% colloidal silicon dioxide and 1 to 1.5 wt.% magnesium stearate, each based on the total weight of the composition, and wherein the mixture of lactose monohydrate and maize starch contains 85 wt.% lactose monohydrate and 15 wt.% maize starch based on the total weight of the mixture.

In another preferred embodiment the pharmaceutical composition comprises 20 to 30 wt.% active pharmaceutical ingredient, 60 to 70 wt.% isomalt, 5 to 6 wt.% crosslinked polyvinylpyrrolidone, 1 to 1.5 wt.% colloidal silicon dioxide and 1 to 1.5 wt.% magnesium stearate, each based on the total weight of the composition.

The present invention also related to any combination of the aforementioned embodiments.

### Examples

The invention is further illustrated by the following examples that are not constructed as being limiting. The amounts of ingredients in the following tables are given in mg per single tablet.

### Example 1

| **ingredient** | **amount** |
|---|---|
| Dasatinib anhydrate | 20 |
| spray dried mixture of 85% lactose monohydrate + 15% maize starch (StarlaC™) | 50 |
| crosslinked polyvinylpyrrolidone (Kollidon™ CL) | 4.5 |
| colloidal silicon dioxide (Aerosil™ 200) | 1.0 |
| magnesium stearate | 1.0 |

Dasatinib anhydrate, Starlc, and Kollidon™ CL are sieved and mixed. After adding the magnesium stearate the powder is mixed again and compressed to tablets, which were subsequently coated with 3 mg Pharmacoat 603 per single tablet.

### Example 2

| **ingredient** | **amount** |
|---|---|
| Dasatinib anhydrate | 20 |
| Isomalt (GalenIQ™ 721) | 50 |
| crosslinked polyvinylpyrrolidone | 4.5 |
| colloidal silicon dioxide (Aerosil™ 200) | 1.0 |
| magnesium stearate | 1.0 |

Dasatinib anhydrate, Starlac™, and Kollidon ™ CL are sieved and mixed. After adding the magnesium stearate the powder is mixed again and compressed to tablets, which were subsequently coated with 3 mg Pharmacoat 603 per single tablet.

### Example 3

| **ingredient** | **amount** |
|---|---|
| Dasatinib hydrochloride | 21.9 |
| spray dried mixture of 85% lactose monohydrate + 15% maize starch (Starlac™) | 50 |
| crosslinked polyvinylpyrrolidone | 4.5 |
| colloidal silicon dioxide (Aerosil™ 200) | 1.0 |
| magnesium stearate | 1.0 |

Dasatinib hydrochloride, Starlac™, and Kollidon ™ CL are sieved and mixed. After adding the magnesium stearate the powder is mixed again and compressed to tablets, which were subsequently coated with 3 mg Pharmacoat 603 per single tablet.

### Example 4

| **ingredient** | **amount** |
|---|---|
| Dasatinib hydrochloride | 21.9 |
| Isomalt (GalenlQ™ 721) | 50 |
| crosslinked polyvinylpyrrolidone | 4.5 |
| colloidal silicon dioxide (Aerosil™ 200) | 1.0 |
| magnesium stearate | 1.0 |

Dasatinib hydrochloride, Starlac™, and GalenlQ™ 721 are sieved and mixed. After adding the magnesium stearate the powder is mixed again and compressed to tablets, which were subsequently coated with 3 mg Pharmacoat 603 per single tablet.

### Description of the figures

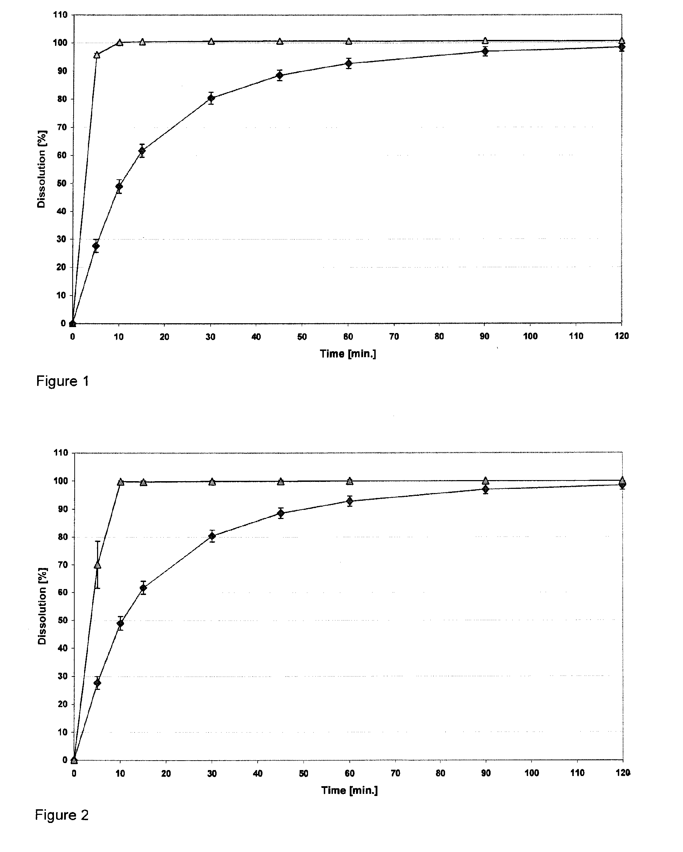

The figures are dissolution profiles obtained from dissolution experiments performed in 900 ml acetate buffer pH 4.0 at 37°C and 75 rpm in a USP paddle apparatus type II. The dissolution of the tablets according to the invention is faster than that of the reference product Spryce™
Figure 1 is the dissolution profile of the pharmaceutical composition obtained according to example 1 (triangles) in comparison with Sprycel™ tablets 20 mg (rhombi).
Figure 2 is the dissolution profile of the pharmaceutical composition obtained according to example 2 (triangles) in comparison with Sprycel™ tablets 20 mg (rhombi).

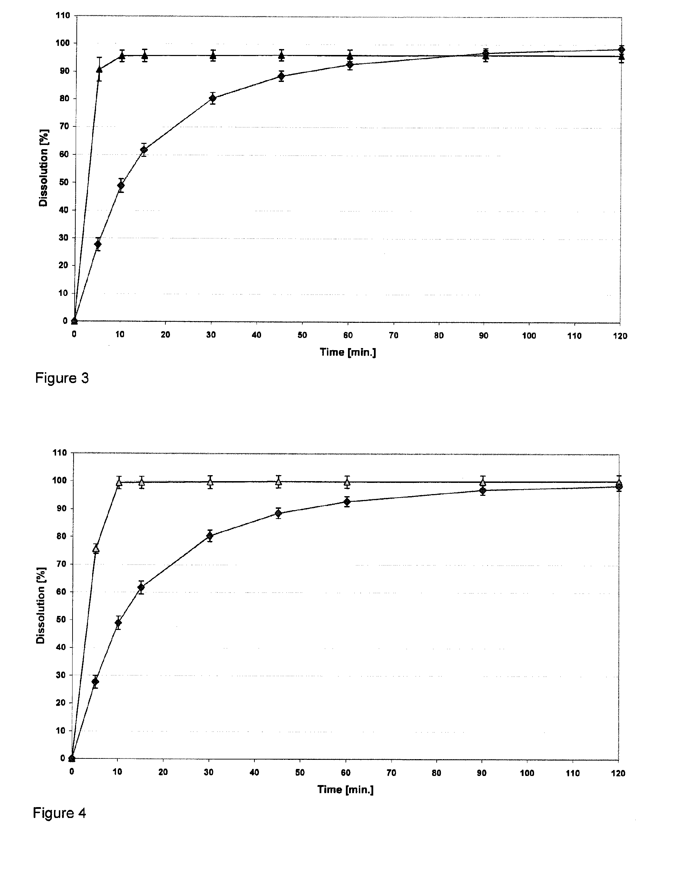

Figure 3 is the dissolution profile of the pharmaceutical composition obtained according to example 3 (triangles) in comparison with Sprycel™ tablets 20 mg (rhombi).
Figure 4 is the dissolution profile of the pharmaceutical composition obtained according to example 4 (triangles) in comparison with Sprycel™ tablets 20 mg (rhombi).

## Claims

1. Pharmaceutical composition in the form of a tablet comprising Dasatinib anhydrate, Dasatinib hydrochloride or Dasatinib hemi ethanol solvate as active pharmaceutical ingredient, wherein the tablet is obtainable by direct compression.

2. The pharmaceutical composition according to claim 1, wherein the composition comprises 20 +/-2 mg, 50 +/-5 mg or 70 +/-7 mg active pharmaceutical ingredient based on the weight of the free base in its unsolvated form.

3. The pharmaceutical composition according to claim 1 or 2, wherein the active pharmaceutical ingredient is present in the pharmaceutical composition in an amount of 10 to 70 wt.%, preferably 20 to 50 wt.% and more preferably 20 to 30 wt.%, each based on the total weight of the composition.

4. The pharmaceutical composition according to any of claims 1 to 3, wherein the mean particle size of the active pharmaceutical ingredient is 1 to 300 µm, preferably 1 to 100 and more preferably 5 to 50 µm.

5. The pharmaceutical composition according to any of claims 1 to 4, wherein the bulk densitiy of the mixture of ingredients prior to direct compression is 0.3 to 0.9 g/ml, preferably 0.4 to 0.8 g/ml.

6. The pharmaceutical composition according to any of claims 1 to 5, wherein the Hausner factor of the mixture of ingredients prior to direct compression is 1.05 to 1.65, preferably 1.1 to 1.5.

7. The pharmaceutical composition according to any of claims 1 to 6 comprising 20 to 30 wt.% of the active pharmaceutical ingredient, 60 to 70 wt.% of a spray dried mixture of lactose monohydrate and maize starch, 5 to 6 wt.% crosslinked polyvinylpyrrolidone, 1 to 1.5 wt. % colloidal silicon dioxide and 1 to 1.5 wt.% magnesium stearate, each based on the total weight of the composition, and wherein the mixture of lactose monohydrate and maize starch contains 85 wt.% lactose monohydrate and 15 wt.% maize starch based on the total weight of the mixture.

8. The pharmaceutical composition according to any of claims 1 to 6 comprising 20 to 30 wt.% active pharmaceutical ingredient, 60 to 70 wt.% isomalt, 5 to 6 wt.% crosslinked polyvinylpyrrolidone, 1 to 1.5 wt.% colloidal silicon dioxide and 1 to 1.5 wt.% magnesium stearate, each based on the total weight of the composition.

9. Method for the preparation of a pharmaceutical composition according any of claims 1 to 8, comprising the steps of mixing the active pharmaceutical ingredient with the further ingredients and forming a tablet from that mixture by direct compression.
